# EUROPEAN PATENT SPECIFICATION

(11) **EP 3 060 152 B1**
(45) Date of publication and mention of the grant of the patent: **25.09.2019**
(21) Application number: 14792983.0
(22) Date of filing: 23.10.2014
(51) Int. Cl.: A61B 18/14

(54) **CATHETER APPARATUSES FOR MODULATION OF NERVES IN COMMUNICATION WITH THE PULMONARY SYSTEM AND ASSOCIATED SYSTEMS**
KATHETERVORRICHTUNGEN ZUR NERVENMODULATION IN VERBINDUNG MIT DEM LUNGENSYSTEM SOWIE ENTSPRECHENDE SYSTEME
CATHÉTER POUR LA MODULATION DE NERFS EN COMMUNICATION AVEC LE SYSTÈME PULMONAIRE ET SYSTÈMES ASSOCIÉS

(30) Priority: 24.10.2013 US 201361961874 P; 24.10.2013 US 201361961873 P; 24.10.2013 US 201361895297 P; 12.09.2014 US 201462049424 P
(43) Date of publication of application: 31.08.2016
(73) Proprietor: Medtronic Ardian Luxembourg S.à.r.l., 2124 Luxembourg (LU)
(72) Inventor: ROTHMAN, Martin, Santa Rosa, CA 95403 (US); GOSHGARIAN, Justin, Santa Rosa, CA 95405 (US); CHANG, William, Santa Rosa, CA 95404 (US)
(74) Representative: Zimmermann & Partner Patentanwälte mbB
(86) International application number: PCT/US2014/062060
(87) International publication number: WO 2015/061624

(56) References cited:
- US-A1- 2010 023 088
- US-A1- 2012 116 382

## Description

### TECHNICAL FIELD

The present technology relates generally to modulation of nerves that communicate with the pulmonary system (e.g., pulmonary artery neuromodulation or "PAN") and associated systems and methods. In particular, several embodiments are directed to radio frequency ("RF") ablation catheter apparatuses for intravascular modulation of nerves that communicate with the pulmonary system and associated systems and methods The invention is set out in the appended claims.

### BACKGROUND

Pulmonary hypertension is an increase in blood pressure in the pulmonary vasculature. When portions of the pulmonary vasculature are narrowed, blocked, or destroyed, it becomes harder for blood to flow through the lungs. As a result, pressure within the lungs increases and makes it hard for the heart to push blood through the pulmonary arteries and into the lungs, thereby causing the pressure in the arteries to rise. Also, because the heart is working harder than normal, the right ventricle becomes strained and weak, which can lead to heart failure. While there are pharmacologic strategies to treat pulmonary hypertension, there is a strong public-health need for alternative treatment strategies.

US 2010/0023088 A1 relates to a system and method for transvascularly stimulating contents of the carotid sheath, wherein in one example, electrodes anchored in the pulmonary artery may be used to simulate neurological targets associated with baroreceptors of the aortic arch.

### BRIEF DESCRIPTION OF THE DRAWINGS

Many aspects of the present technology can be better understood with reference to the following drawings. The components in the drawings are not necessarily to scale. Instead, emphasis is placed on illustrating clearly the principles of the present technology.
FIG. 1 is a partially-schematic view of a neuromodulation system configured in accordance with an embodiment of the present technology.
FIG. 2A is an enlarged side view illustrating a therapeutic assembly of the catheter of FIG. 1 in a low-profile configuration configured in accordance with an embodiment of the present technology.
FIG. 2B is a further enlarged cut-away view of a portion of the therapeutic assembly of FIG. 2A configured in accordance with an embodiment of the present technology.
FIG. 2C is a cross-sectional end view taken along line 2C-2C in FIG. 2A.
FIG. 3A is an illustrative cross-sectional anatomical front view showing the advancement of the catheter shown in FIG. 1 along an intravascular path in accordance with an embodiment of the present technology.
FIG. 3B is a side view of the therapeutic assembly shown in FIG. 2A within the main pulmonary artery in a low-profile configuration configured in accordance with an embodiment of the present technology.
FIG. 3C is a side view of the therapeutic assembly shown in FIG. 2A within the main pulmonary artery in a deployed configuration configured in accordance with an embodiment of the present technology.
FIG. 3D is a side view of the therapeutic assembly shown in FIG. 2A within the left pulmonary artery in a deployed configuration configured in accordance with an embodiment of the present technology.
FIG. 3E is a side view of the therapeutic assembly shown in FIG. 2A within the right pulmonary artery in a deployed configuration configured in accordance with an embodiment of the present technology.
FIG. 4 is an illustrative cross-sectional anatomical front view showing the advancement of the catheter shown in FIG. 1 along another intravascular path in accordance with an embodiment of the present technology.
FIG. 5 is a side view of a therapeutic assembly having a single wire electrode configured in accordance with an embodiment of the present technology.
FIGS. 6A-6B are schematic representations illustrating rotation of the therapeutic assembly.
FIG. 7 is a schematic side view of a catheter having an inner sheath configured in accordance with an embodiment of the present technology.
FIGS. 8A-8B are side views of a catheter having an inner sheath positioned within the left pulmonary artery configured in accordance with an embodiment of the present technology.
FIG. 9 is a side view of a therapeutic assembly in a deployed configuration having an anchoring collar positioned within the left pulmonary artery configured in accordance with an embodiment of the present technology.
FIG. 10 is a side view of a therapeutic assembly in a deployed configuration having an anchoring collar positioned within the left pulmonary artery configured in accordance with an embodiment of the present technology.
FIG. 11 is a side view of a therapeutic assembly having an anchoring device (shown in cross-section) within the right pulmonary artery in a deployed configuration configured in accordance with an embodiment of the present technology.
FIG. 12 is a side view of a therapeutic assembly having an anchoring device within the right pulmonary artery in a deployed configuration configured in accordance with an embodiment of the present technology.
FIG. 13 is a side view of a therapeutic assembly having an extendable shaft within the left pulmonary artery in a deployed configuration configured in accordance with an embodiment of the present technology.
FIG. 14 is a side view of a therapeutic assembly mechanically isolated from the shaft within the right pulmonary artery in a deployed configuration configured in accordance with an embodiment of the present technology.
FIG. 15 is a side view of therapeutic assemblies in a deployed configuration configured in accordance with an embodiment of the present technology.
FIG. 16 is a side view of a therapeutic assembly having an inflection section in a deployed configuration configured in accordance with an embodiment of the present technology.

### DETAILED DESCRIPTION

The present technology is directed to neuromodulation devices and associated systems and methods. Some embodiments of the present technology, for example, are directed to catheters, catheter systems, and methods for modulation of nerves that communicate with the pulmonary system. At least some of these nerves may be partially or completely incapacitated or otherwise effectively disrupted.

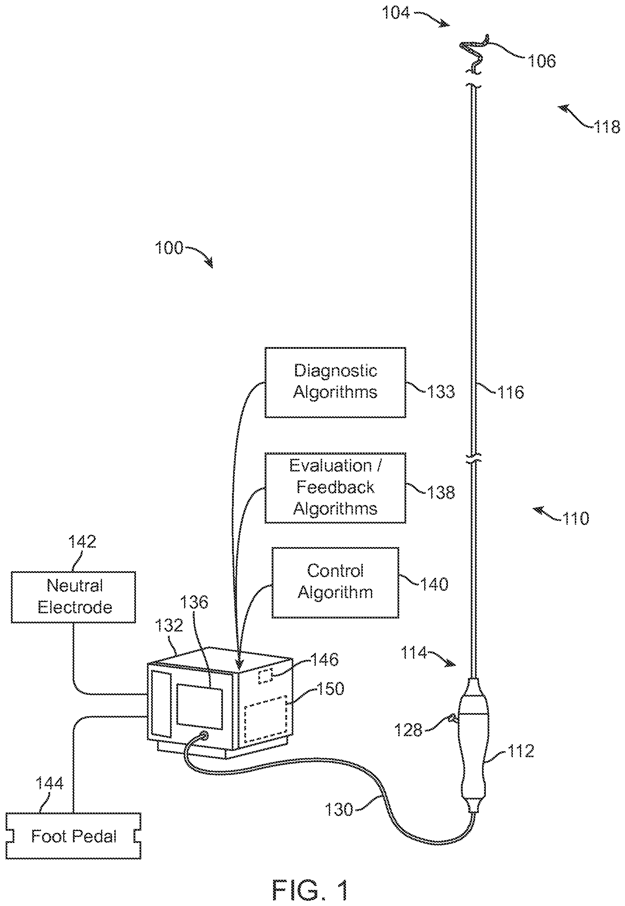

Catheters, catheter systems, and methods described herein may be used, for example, for PAN. PAN is the partial or complete incapacitation or other effective disruption of nerves innervating the pulmonary arteries. Specific details of several embodiments of the technology are described below with reference to FIGS. 1-3E. Although many of the embodiments are described below with respect to systems, devices, and methods for PAN, the technology is applicable to other applications such as modulation of other nerves that communicate with the pulmonary system, modulation of peripheral nerves, and/or treatments other than neuromodulation. Moreover, as further described herein, while the technology may be used in helical or spiral neuromodulation devices, it may also be used in non-helical or non-spiral neuromodulation devices as appropriate. Furthermore, other embodiments in addition to those described herein are within the scope of the technology. Additionally, several other embodiments of the technology can have different configurations, components, or procedures than those described herein. A person of ordinary skill in the art, therefore, will accordingly understand that the technology can have other embodiments with additional elements, or the technology can have other embodiments without several of the features shown and described below with reference to FIGS. 1-16.

As used herein, the terms "distal" and "proximal" define a position or direction with respect to the treating clinician or clinician's control device (e.g., a handle assembly). "Distal" or "distally" are a position distant from or in a direction away from the clinician or clinician's control device. "Proximal" and "proximally" are a position near or in a direction toward the clinician or clinician's control device.

### I. Pulmonary Artery Neuromodulation

As used herein, "pulmonary vessel(s)" include any blood vessel that is adjacent to and/or provides intravascular access proximate to neural pathways that communicate with the pulmonary system. Examples of pulmonary vessels include pulmonary arteries, such as the main pulmonary artery ("MPA"), the right pulmonary artery ("RPA"), the left pulmonary artery ("LPA"), segmental pulmonary arteries, and sub-segmental pulmonary arteries. In some embodiments, the bifurcated portion of a pulmonary artery may be neuromodulated using methods and/or devices described herein. Other non-limiting examples of pulmonary vessels include the right ventricular outflow tract, pulmonary arterioles, and/or any branch and/or extension of any of the pulmonary vessels described above. Neuromodulation may take place in and/or near one or more pulmonary vessels, such as in and/or near one or more pulmonary arteries. In some embodiments, neuromodulation may take place in a distal portion of the main pulmonary artery and/or in one or more branches (e.g., distal branches) of the main pulmonary artery. In certain embodiments, neuromodulation may be effected at or near the pulmonary valve (e.g., to affect nerves above and/or below the pulmonary valve). Methods and/or devices described herein may be used to neuromodulate any suitable pulmonary vessels or other target sites. Although many embodiments are described for use in a pulmonary arterial approach, it is also possible to use the technology in a pulmonary venous approach, or even in a non-vascular approach, such as a cutaneous and/or transcutaneous approach to the nerves that innervate the pulmonary system. For example, the vagal and phrenic nerves may lie outside the lungs (e.g., in the neck region and/or in the inlet to the thoracic cavity) at various locations that may render them amenable to access via cutaneous puncture or to transcutaneous denervation. As such, devices and/or methods described herein may be used to effect modulation of vagal and/or phrenic nerves from within a carotid vein and/or a jugular vein. Neuromodulation at one or both of those locations may be effective (e.g., may provide a therapeutically beneficial effect with respect to treating pulmonary hypertension).

PAN comprises inhibiting, reducing, and/or blocking neural communication along neural fibers (i.e., efferent and/or afferent nerve fibers) innervating the pulmonary vessels. Such incapacitation (both from PAN and from modulation of other neural pathways that communicate with the pulmonary system) can be long-term (e.g., permanent or for periods of months, years, or decades) or short-term (e.g., for periods of minutes, hours, days, or weeks). PAN and modulation of other neural pathways that communicate with the pulmonary system are expected to efficaciously treat pulmonary hypertension. Subjects with pulmonary hypertension generally have high blood pressure in the lung vasculature that may lead to heart failure and they may, for example, experience symptoms such as dyspnea (shortness of breath), syncope, fatigue, chest pain and/or edema, and/or other symptoms as well. Neuromodulation using methods and/or devices described here may provide a therapeutically beneficial reduction in one or more of these symptoms.

Various techniques can be used to partially or completely incapacitate neural pathways, such as those innervating the pulmonary vessels. The purposeful application of energy (e.g., electrical energy, thermal energy, etc.) to tissue by energy delivery element(s) can induce one or more desired thermal heating effects on localized regions of the pulmonary vessels and nerves along or otherwise near the pulmonary vessels. The energy may be selected to effect the desired neuromodulation (e.g., denervation). Such nerves include, for example, nerves which lay intimately within or adjacent to the adventitia of the pulmonary vessels. The purposeful application of the thermal heating effects can achieve neuromodulation along all or a portion of the nerves.

It is typically advantageous to at least generally maintain the position of a neuromodulation unit relative to the surrounding anatomy during a neuromodulation treatment. For example, it can be advantageous to at least generally maintain stable contact between a therapeutic element of a neuromodulation unit and an inner wall of a body lumen (e.g., a blood vessel, a duct, an airway, or another naturally occurring lumen within the human body) during a neuromodulation treatment. This can enhance control and/or monitoring of the treatment, reduce trauma to the body lumen, and/or have other advantages. In some cases, at least generally maintaining the position of a neuromodulation unit relative to the target anatomy during a neuromodulation treatment can be challenging. For example, certain organs body tissues may move in response to respiration, cardiac contraction and relaxation, peristaltic movement within blood vessels, and patient movement. Such movement of organs and other tissues in a patient's body can cause movement of a catheter shaft within a vessel or other disadvantageous relative movement between a neuromodulation unit connected to the shaft and the anatomy at a target site. Moreover, the anatomy itself may present difficulties to maintaining a device at the target site. For example, a pulmonary artery may generally be tapered, which can make it difficult to securely deploy certain device configurations in that location.

Another difficulty may exist with respect to initial positioning of a neuromodulation unit. When a neuromodulation unit is initially positioned at a treatment location within a pulmonary vessel or other body lumen (e.g., a renal vessel), the position of the neuromodulation unit may be suboptimal. For example, a catheter and/or a sheath carrying the catheter may be insufficiently flexible to match the curvature of anatomy near the treatment location (e.g., the curvature of a pulmonary artery between the MPA and the RPA and/or LPA). This may cause the catheter and/or the sheath to enter the body lumen out of alignment with a longitudinal dimension or other feature of the body lumen. When a neuromodulation unit of a misaligned catheter is initially moved into an expanded form, the neuromodulation unit may also not be aligned with the body lumen. When a neuromodulation unit is misaligned, one or more therapeutic elements of the neuromodulation unit may be out of contact or in poor contact with an inner wall of a body lumen, thereby resulting in suboptimal (or no) energy delivery to a target site. Even when the neuromodulation unit is sufficiently well aligned for treatment to begin, misalignment and migration may occur later and disturb the wall contact, potentially requiring the treatment to be aborted. Correcting misalignment of a neuromodulation unit can be challenging when the neuromodulation unit remains directly attached to an associated shaft trapped at a sharp turn.

### II. Selected Embodiments of Catheter Apparatuses

FIG. 1 is partially-schematic diagram illustrating a pulmonary neuromodulation system 100 ("system 100") configured in accordance with an embodiment of the present technology. The system 100 includes an intravascular catheter 110 operably coupled to an energy source or energy generator 132 via a connector 130 (e.g., a cable). The catheter 110 can include an elongated shaft 116 having a proximal portion 114 and a distal portion 118. The catheter 110 also includes a handle assembly 112 at the proximal portion 114. The catheter 110 can further include a therapeutic assembly 104 carried by or affixed to the distal portion 118 of the elongated shaft 116, and the therapeutic assembly 104 can have one or more energy delivery elements 106 configured to modulate nerves at or near the treatment location. The elongated shaft 116 can be configured to intravascularly locate the therapeutic assembly 104 at a treatment location within a pulmonary artery, renal artery, or other blood vessel, or in a non-vascular delivery, such as through a ureter or other naturally occurring body lumen of a human patient (for example, via a natural orifice transluminal endoscopic surgery (NOTES) procedure). In certain embodiments, an extracorporeal approach may be employed, such as by using extracorporeal ultrasound.

The energy generator 132 can be configured to generate a selected form and/or magnitude of energy for delivery to the treatment site via the energy delivery elements 106 of the therapeutic assembly 104. For example, the energy generator 132 can include an energy source (not shown) configured to generate RF energy (e.g., monopolar and/or bipolar, pulsed and/or non-pulsed, intravascular or extravascular, etc.), microwave energy, optical energy, ultrasound energy (e.g., intravascularly delivered ultrasound, extracorporeal ultrasound, high-intensity focused ultrasound (HIFU), etc.), direct heat energy, radiation (e.g., infrared, visible, gamma, etc.), or another suitable type of energy. For embodiments having multiple energy delivery elements 106, energy can be delivered to all or a portion of the energy delivery elements 106 simultaneously or at different times. Different firing sequences may be used, as appropriate. As an example, a single energy delivery element may be selected at a time, or a combination of certain energy delivery elements, or all energy delivery elements. Energy delivery elements may be fired sequentially or simultaneously and/or may be fired according to a particular algorithm and/or operator input. In some embodiments, the therapeutic assembly 104 and/or energy delivery elements 106 can be configured for use with a source of cryotherapeutic energy, and/or for use with a source of one or more chemicals, such as drugs or other agents (e.g., to provide the cryotherapeutic energy and/or chemical(s) to a target site for PAN). It is believed that cryotherapeutic energy, for example, may be especially effective for PAN, where air within the lungs may function as a heat sink. Cryotherapeutic energy may provide a relatively deep and/or uniform freezing of tissue.

In some embodiments, instead of or in addition to the energy delivery elements 106, the therapeutic assembly 104 can include one or more substance delivery features (e.g., ports) to produce chemically based neuromodulation by delivering one or more chemicals (e.g., guanethidine, ethanol, phenol, a neurotoxin (e.g., vincristine)), and/or other suitable agents selected to alter, damage, or disrupt nerves. For example, in some embodiments the therapeutic assembly 104 can include one or more puncture elements or needles (not shown) having one or more inlet ports. The puncture elements can be configured, when deployed, to extend from the therapeutic assembly 104 into the vessel wall at the treatment site to deliver one or more chemicals. In some embodiments, one or more puncture elements may be deployed and/or positioned using x-ray fluoroscopy. In certain embodiments, the therapeutic assembly 104 can include at least one expandable element (not shown), such as a balloon, a basket, or a wire cage, that is configured to carry one or more chemicals and release the chemical(s) once the expandable element is expanded and in apposition with the vessel wall. For example, in some embodiments a radially exterior surface of the expandable element can be coated with selected chemical(s). In yet other embodiments, the expandable element can be configured to release the chemical(s) from an interior portion of the expandable element when submitted to a predetermined force threshold (e.g., radial forces exerted by the vessel walls).

Furthermore, the energy generator 132 can be configured to control, monitor, supply, or otherwise support operation of the catheter 110. For example, a control mechanism, such as foot pedal 144, may be connected (e.g., pneumatically connected or electrically connected) to the energy generator 132 to allow an operator to initiate, terminate and/or adjust various operational characteristics of the energy generator, such as power delivery. In some embodiments, the energy generator 132 may be configured to provide delivery of a monopolar electric field via the energy delivery element(s) 106. For example, in some bipolar embodiments, the distal portion 118 of the shaft could include a ground electrode insulated from the rest of the distal portion 118. In such embodiments, a neutral or dispersive electrode 142 may be electrically connected to the energy generator 132 and attached to the exterior of the patient (not shown). It can be advantageous to position the dispersive electrode such that it does not interfere with the line of sight of the imaging device.

In some embodiments, the system 100 includes a remote control device (not shown) that can be configured to be sterilized to facilitate its use within a sterile field. The remote control device can be configured to control operation of the therapeutic assembly 104, the energy generator 132, and/or other suitable components of the system 100. For example, the remote control device can be configured to allow for selective activation of the therapeutic assembly 104. In other embodiments, the remote control device may be omitted and its functionality may be incorporated into the handle 112 or energy generator 132.

As shown in FIG. 1, the energy generator 132 can further include an indicator or display screen 136. The energy generator 132 can include other indicators, including one or more LEDs, a device configured to produce an audible indication, and/or other suitable communicative devices. In the embodiment shown in FIG. 1, the display 136 includes a user interface configured to receive information or instructions from a user and/or provide feedback to the user. For example, the energy generator 132 can be configured to provide feedback to an operator before, during, and/or after a treatment procedure via the display 136. The feedback can be based on output from one or sensors (not shown) associated with the therapeutic assembly 104 such as temperature sensor(s), impedance sensor(s), current sensor(s), voltage sensor(s), flow sensor(s), chemical sensor(s), ultrasound sensor(s), optical sensor(s), pressure sensor(s) and/or other sensing devices.

The system 100 can further include a controller 146 having, for example, memory (not shown) and processing circuitry (not shown). The memory and storage devices are computer-readable storage media that may be encoded with non-transitory, computer-executable instructions such as diagnostic algorithm(s) 133, control algorithm(s) 140, and/or evaluation/feedback algorithm(s) 138. The control algorithms 140 can be executed on a processor (not shown) of the system 100 to control energy delivery to the energy delivery elements 106. In some embodiments, selection of one or more parameters of an automated control algorithm 140 for a particular patient may be guided by diagnostic algorithms 133 that measure and evaluate one or more operating parameters prior to energy delivery. The diagnostic algorithms 133 provide patient-specific feedback to the clinician prior to activating the energy delivery elements 106 which can be used to select an appropriate control algorithm 140 and/or modify the control algorithm 140 to increase the likelihood of efficacious neuromodulation.

Although in the embodiment shown in FIG. 1 the controller 146 is incorporated into the energy generator 132, in other embodiments the controller 146 may be a separate component distinct from the energy generator 132. For example, additionally or alternatively, the controller 146 can be a personal computer(s), server computer(s), handheld or laptop device(s), multiprocessor system(s), microprocessor-based system(s), programmable consumer electronic(s), digital camera(s), network PC(s), minicomputer(s), mainframe computer(s), and/or any suitable computing environment.

In some embodiments, the energy source 132 may include a pump 150 or other suitable pressure source (e.g., a syringe) operably coupled to an irrigation port (not shown) at the distal portion 118 of the catheter 110. In other embodiments, the pump 150 can be a standalone device separate from the energy source 132. Positive pressure generated by the pump 150 can be used, for example, to push a protective agent (e.g., saline) through the irrigation port to the treatment site. In yet other embodiments, the catheter 110 can include an adapter (not shown) (e.g., a luer lock) configured to be operably coupled to a syringe (not shown) and the syringe can be used to apply pressure to the shaft 116.

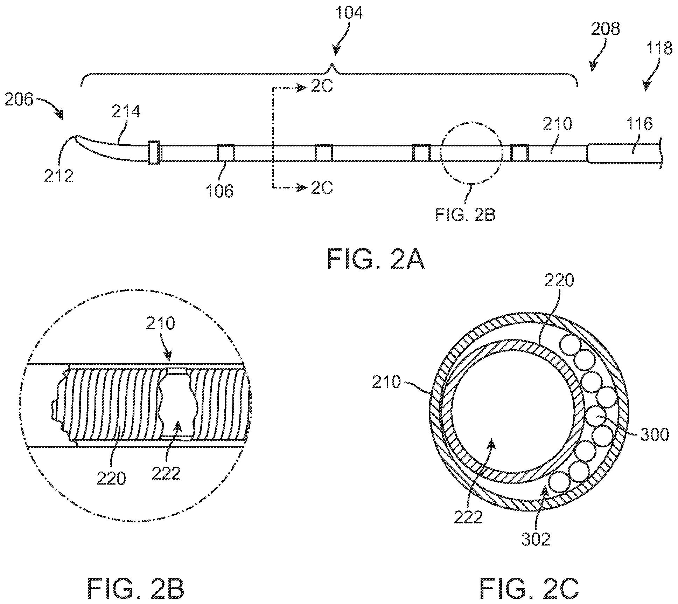

FIG. 2A is a side view of the therapeutic assembly 104 in a low-profile or delivery state in accordance with an embodiment of the present technology. A proximal region 208 of the therapeutic assembly 104 can be carried by or affixed to the distal portion 118 of the elongated shaft 116. For example, all or a portion (e.g., a proximal portion) of the therapeutic assembly 104 can be an integral extension of the shaft 116. A distal region 206 of the therapeutic assembly 104 may terminate distally with, for example, an atraumatic, flexible curved tip 214 having an opening 212 at its distal end. In some embodiments, the distal region 206 of the therapeutic assembly 104 may also be configured to engage another element of the system 100 or catheter 110.

FIG. 2B is an enlarged view of a portion of the therapeutic assembly 104 of FIG. 2A, and FIG. 2C is a cross-sectional end view taken along line 2C-2C in FIG. 2A. Referring to FIGS. 2A-2C together, the therapeutic assembly 104 can include the one or more energy delivery elements 106 carried by a helical/spiral-shaped support structure 210. The helical/spiral support structure 210 can have one or more turns (e.g., two turns, etc.). The energy delivery elements 106 can be RF electrodes, ultrasound transducers, cryotherapeutic cooling assemblies, direct heat elements or other therapeutic delivery elements. The energy delivery elements 106, for example, can be separate band electrodes axially spaced apart along the support structure 210 (e.g., adhesively bonded, welded (e.g., laser bonded) or bonded by mechanical interference to the support structure 210 at different positions along the length of the support structure 210). In other embodiments, the therapeutic assembly 104 may have a single energy delivery element 106 at or near the distal portion 118 of the shaft 116.

In embodiments including where the support structure 210 includes more than one energy delivery element 106, the support structure 210 can include, for example, between 1 and 12 energy delivery elements (e.g., 1 energy delivery element, 4 energy delivery elements, 10 energy delivery elements, 12 energy delivery elements, etc.). In particular embodiments, the therapeutic assembly 104 can include an even number of energy delivery elements 106. In some embodiments, the energy delivery elements 106 can be spaced apart along the support structure 210 every 1 mm to 50 mm, such as every 2 mm to every 15 mm (e.g., every 10 mm, etc.). In the deployed configuration, the support structure 210 and/or therapeutic assembly 104 can have an outer diameter between about 12 mm and about 20 mm (e.g., between about 15 mm and about 18 mm). Additionally, the support structure 210 and energy delivery elements 106 can be configured for delivery within a guide catheter between 5 Fr and 9 Fr. In other examples, other suitable guide catheters may be used, and outer dimensions and/or arrangements of the catheter 110 can vary accordingly.

In some embodiments, the energy delivery elements 106 are formed from a metal, such as gold, platinum, alloys of platinum and iridium or other suitable electrically conductive materials. The number, arrangement, shape (e.g., spiral and/or coil electrodes) and/or composition of the energy delivery elements 106 may vary. Each of the individual energy delivery elements 106 can be electrically connected to the energy generator 132 by a conductor or bifilar wire 300 (FIG. 2C) extending through a lumen 302 (FIG. 2C) of the shaft 116 and/or support structure 210. For example, the individual energy delivery elements 106 may be welded or otherwise electrically coupled to corresponding energy supply wires 300, and the wires 300 can extend through the elongated shaft 116 for the entire length of the shaft 116 such that proximal ends of the wires 300 are coupled to the handle 112 and/or to the energy generator 132.

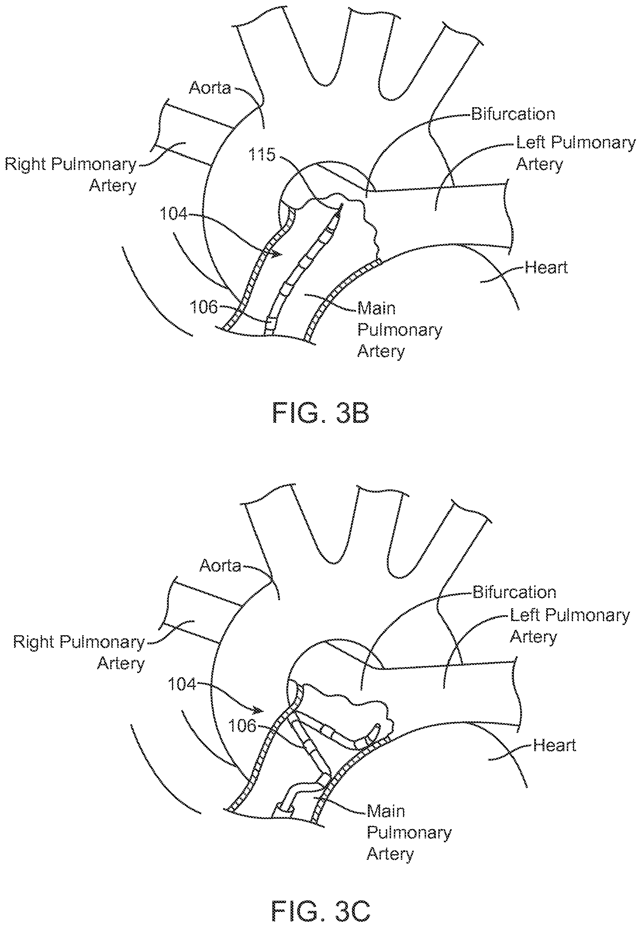

As shown in the enlarged cut-away view of FIG. 2B, the support structure 210 can be a tube (e.g., a flexible tube) and the therapeutic assembly 104 can include a pre-shaped control member 220 positioned within the tube. Upon deployment, the control member 220 can form at least a portion of the therapeutic assembly 104 into a deployed state (FIG. 3C-3E). For example, the control member 220 can have a pre-set configuration that gives at least a portion of the therapeutic assembly 104 a helical/spiral configuration in the deployed state (FIG. 3C-3E). In some embodiments, the control member 220 includes a tubular structure comprising a Nitinol multifilar stranded wire with a lumen 222 therethrough and sold under the trademark HELICAL HOLLOW STRAND™ (HHS), and commercially available from Fort Wayne Metals of Fort Wayne, Indiana. The lumen 222 can define a passageway for receiving a guide wire (not shown) that extends proximally from the opening 212 (FIG. 2A) at the tip 214 of the therapeutic assembly 104. In other embodiments, the control member 220 may be composed of different materials and/or have a different configuration. For example, the control member 220 may be formed from nickel-titanium (Nitinol), shape memory polymers, electro-active polymers or other suitable shape memory materials that are pre-formed or pre-shaped into the desired deployed state. Alternatively, the control member 220 may be formed from multiple materials such as a composite of one or more polymers and metals.

As shown in FIG. 2C, the support structure 210 can be configured to fit tightly against the control member 220 and/or wires 300 to reduce space between an inner portion of the support structure 210 and the components positioned therein. For example, the control member 220 and the inner wall of the support structure 210 can be in intimate contact such that there is little or no space between the control member 220 and the support structure 210. Such an arrangement can help to reduce or prevent the formation of wrinkles in the therapeutic assembly 104 during deployment. The support structure 210 may be composed of one or more polymer materials such as polyamide, polyimide, polyether block amide copolymer sold under the trademark PEBAX®, polyethylene terephthalate (PET), polypropylene, aliphatic, polycarbonate-based thermoplastic polyurethane sold under the trademark CARBOTHANE®, ELASTHANE™ TPU, a polyether ether ketone (PEEK) polymer, or another suitable material that provides sufficient flexibility to the support structure 210.

In some embodiments, when the therapeutic assembly 104 and/or support structure 210 is in deployed configuration, the therapeutic assembly 104 and/or support structure 210 preferably define a minimum width of greater than or equal to approximately 0.040 inches. Additionally, the support structure 210 and energy delivery elements 106 are configured for delivery within a guide catheter no smaller than a 5 French guide catheter. In other examples, other suitable guide catheters may be used, and outer dimensions and/or arrangements of the catheter 110 can vary accordingly.

Referring to FIG. 2A, the curved tip 214 can be configured to provide an exit (e.g., via the opening 212) for a guide wire that directs the guide wire away from a wall of a vessel or lumen at or near a treatment location. As a result, the curved tip 214 can facilitate alignment of the therapeutic assembly 104 in the vessel or lumen as it expands from the delivery state shown in FIG. 2A. Furthermore, the curved tip 214 can reduce the risk of injuring a wall of the vessel or lumen when a distal end of a guide wire is advanced from the opening 212. The curvature of the tip 214 can be varied depending upon the particular sizing/configuration of the therapeutic assembly 104 and/or anatomy at a treatment location. In some embodiments, the tip 214 may also comprise a radiopaque marker and/or one or more sensors (not shown) positioned anywhere along the length of the tip. For example, in some embodiments, the tip can include one or more layers of material (e.g., the same or different materials) and the radiopaque marker can be sandwiched between two or more layers. Alternatively, the radiopaque marker can be soldered, glued, laminated, or mechanically locked to the exterior surface of the tip 214. In other embodiments, the entire tip 214 can be made of a radiopaque material. The tip 214 can be affixed to the distal end of the support structure 210 via adhesive, crimping, over-molding, or other suitable techniques.

The flexible curved tip 214 can be made from a polymer material (e.g., polyether block amide copolymer sold under the trademark PEBAX™), a thermoplastic polyether urethane material (e.g., sold under the trademarks ELASTHANE™ or PELLETHANE®), or other suitable materials having the desired properties, including a selected durometer. As noted above, the tip 214 is configured to provide an opening for the guide wire, and it is desirable that the tip itself maintain a desired shape/configuration during operation. Accordingly, in some embodiments, one or more additional materials may be added to the tip material to help improve tip shape retention. In one particular embodiment, for example, about 5 to 30 weight percent of siloxane can be blended with the tip material (e.g., the thermoplastic polyether urethane material), and electron beam or gamma irradiation may be used to induce cross-linking of the materials. In other embodiments, the tip 214 may be formed from different material(s) and/or have a different arrangement. For example, in some embodiments the tip 214 may be straight.

In some embodiments, the distal portion 118 of the catheter can include one or more irrigation ports (not shown) configured to emit one or more protective agents (e.g., saline) before, during, and/or after energy delivery to cool the energy delivery elements and surrounding tissue. The irrigation port(s) may be located anywhere along the support structure 210 and/or distal portion 118 of the shaft 116. The irrigation port(s) can be in fluid connection with one or more corresponding irrigation lumens that extends proximally along the shaft 116 from the irrigation port to the handle 112 and/or energy generator 132. In some embodiments, the catheter can include multiple irrigation ports, all in fluid communication with a corresponding irrigation lumen. In particular embodiments, an irrigation lumen can be coupled to a pump 150 (see FIG. 1) or syringe (not shown) to facilitate conveyance of the protective agent along the irrigation lumen and irrigation of protective agent through the irrigation port(s).

### III. Selected Delivery Embodiments

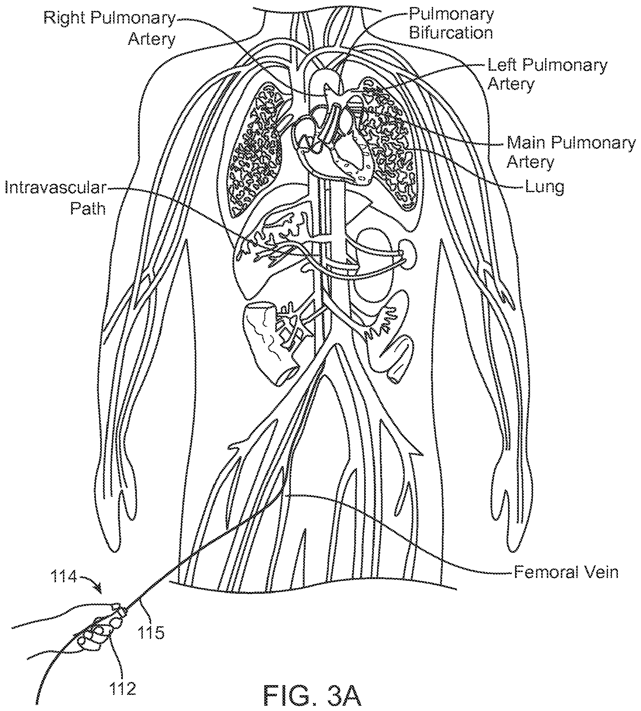

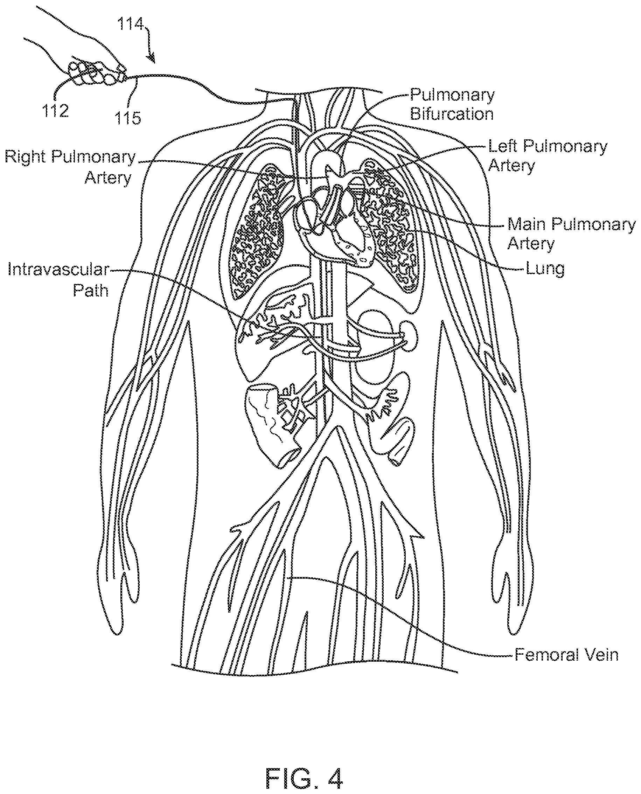

Referring to FIG. 3A, intravascular delivery of the therapeutic assembly 104 can include percutaneously inserting a guide wire 115 within the vasculature at an access site (e.g., femoral (FIG. 3A), brachial, radial, axillary, or subclavian artery or vein (see FIG. 4)) and progressing the guidewire to the MPA. The lumen 222 (FIG. 2C) of the shaft 116 and/or therapeutic assembly 104 can be configured to receive a guide wire 115 in an over-the-wire or rapid exchange configuration. As shown in FIG. 3B, the shaft 116 and the therapeutic assembly 104 (in the delivery state) can then be advanced along the guide wire 115 until at least a portion of the therapeutic assembly 104 reaches the treatment location. As illustrated in FIGS. 3A and 4, a section of the proximal portion 114 of the shaft 116 can be extracorporeally positioned and manipulated by the operator (e.g., via the actuator 128 shown in FIG. 1) to advance the shaft 116 through the intravascular path and remotely manipulate the distal portion 118 of the shaft 116.

Image guidance, e.g., computed tomography (CT), fluoroscopy, intravascular ultrasound (IVUS), optical coherence tomography (OCT), intracardiac echocardiography (ICE), or another suitable guidance modality, or combinations thereof, may be used to aid the clinician's positioning and manipulation of the therapeutic assembly 104. For example, a fluoroscopy system (e.g., including a flat-panel detector, x-ray, or c-arm) can be rotated to accurately visualize and identify the treatment site. In other embodiments, the treatment site can be located using IVUS, OCT, and/or other suitable image mapping modalities that can correlate the treatment site with an identifiable anatomical structure (e.g., a spinal feature) and/or a radiopaque ruler (e.g., positioned under or on the patient) before delivering the catheter 110. Further, in some embodiments, image guidance components (e.g., IVUS, OCT) may be integrated with the catheter 110 and/or run in parallel with the catheter 110 to provide image guidance during positioning of the therapeutic assembly 104. For example, such image guidance components can be coupled to a distal portion of the catheter 110 to provide three-dimensional images of the vasculature proximate the site to facilitate positioning or deploying the therapeutic assembly 104 within the pulmonary blood vessel.

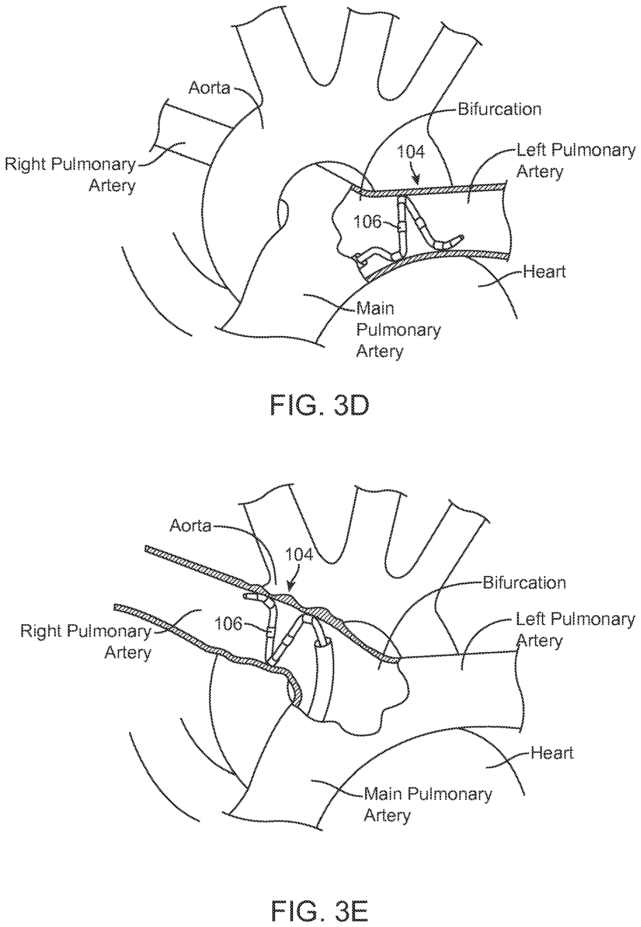

Once the therapeutic assembly 104 is positioned at a treatment location within a pulmonary artery, the guide wire 115 can be at least partially removed (e.g., withdrawn) from or introduced (e.g., inserted) into the therapeutic assembly 104 to transform or otherwise move the therapeutic assembly 104 to a deployed configuration. FIG. 3C is a side view of the therapeutic assembly 104 shown in FIG. 2A within the main pulmonary artery in a deployed configuration, FIG. 3D is a side view of the therapeutic assembly 104 within the left pulmonary artery, and FIG. 3E is a side view of the therapeutic assembly 104 within the right pulmonary artery in accordance with an embodiment of the present technology. As shown in FIGS. 3C-3E, in the deployed state, at least a portion of the therapeutic assembly 104 can be configured to contact an inner wall of a pulmonary artery and to cause a fully-circumferential lesion about a longitudinal axis without the need for repositioning. For example, the therapeutic assembly 104 can be configured to form a lesion or series of lesions (e.g., a helical/spiral lesion or a discontinuous lesion) that is fully-circumferential overall, but generally non-circumferential at longitudinal segments of the treatment location (e.g., spaced longitudinally along the vessel at different circumferential locations). This can facilitate precise and efficient treatment with a low possibility of vessel stenosis. In other embodiments, the therapeutic assembly 104 can be configured to form a partially-circumferential lesion or a fully-circumferential lesion at a single longitudinal segment of the treatment location. In some embodiments, the therapeutic assembly 104 can be configured to cause therapeutically-effective neuromodulation (e.g., using ultrasound energy) without contacting a vessel wall.

As shown in FIGS. 3C-3E, in the deployed state, the therapeutic assembly 104 defines a substantially helical/spiral structure in contact with the pulmonary artery wall along a helical/spiral path. One advantage of this arrangement is that pressure from the helical/spiral structure can be applied to a large range of radial directions without applying pressure to a circumference of the pulmonary vessel. Thus, the spiral/helically-shaped therapeutic assembly 104 is expected to provide stable contact between the energy delivery elements 106 and the pulmonary vessel wall when the wall moves in any direction. Furthermore, pressure applied to the pulmonary vessel wall along a helical/spiral path is less likely to stretch or distend a circumference of a vessel that could thereby cause injury to the vessel tissue. Still another feature of the expanded helical/spiral structure is that it may contact the pulmonary vessel wall in a large range of radial directions and maintain a sufficiently open lumen in the pulmonary vessel allowing blood to flow through the helix/spiral during therapy.

In some procedures it may be necessary to adjust the positioning of the therapeutic assembly 104 one or more times. For example, the therapeutic assembly 104 can be used to modulate nerves proximate the wall of the main pulmonary artery, the left pulmonary artery, and/or the right pulmonary artery and/or any branch or extension, and/or other pulmonary vessels or sites proximate to neural pathways in communication with the pulmonary system. Additionally, in some embodiments the therapeutic assembly 104 may be repositioned within the same pulmonary vessel or at the same site multiple times within the same procedure. After repositioning, the clinician may then re-activate the therapeutic assembly 104 to modulate the nerves.

Although the embodiments shown in FIGS. 3C-3E show a deployed therapeutic assembly 104 in a spiral/helically-shaped configuration, in other embodiments, the therapeutic assembly 104 and/or other portions of the therapeutic assembly 104 can have other suitable shapes, sizes, and/or configurations (e.g., bent, deflected, zig-zag, Malecot, etc.). In some embodiments, for example, the therapeutic assembly 104 can include a non-occlusive expandable structure. Other suitable devices and technologies are described in, for example, U.S. Patent Application No. 12/910,631, filed October 22, 2010, U.S. Patent Application No. 13/279,205, filed October 21, 2011, U.S. Patent Application No. 13/279,330, filed October 23, 2011, U.S. Patent Application No. 13/281,360, filed October 25, 2011, U.S. Patent Application No. 13/281,361, filed October 25, 2011, PCT Application No. PCT/US11/57754, filed October 25, 2011, U.S. Provisional Patent Application No. 61/646,218, filed May 5, 2012, U.S. Patent Application No. 13/793,647, filed March 11, 2013, and U.S. Provisional Patent Application No. 61/961,874, filed October 24, 2013.

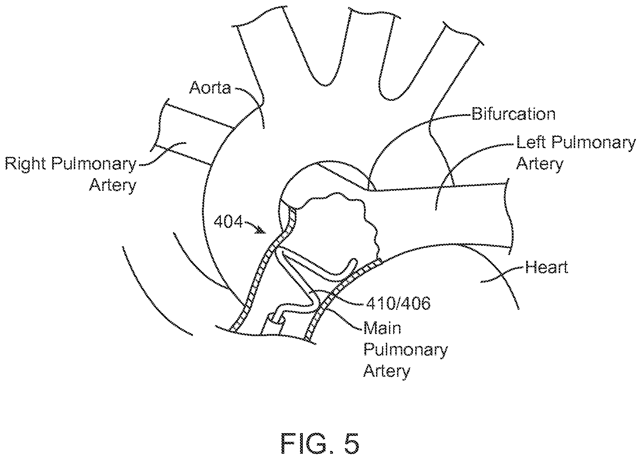

FIG. 5 shows another embodiment of a therapeutic assembly 404 comprising a support structure 410 defined by a single wire electrode 406. For example, the support structure 410 can be a unipolar single metal wire (e.g., Nitinol) that is pre-formed into a helical/spiral shape. The single wire electrode 406 can have a continuous electrically conductive surface along all or a significant part of its length such that it forms a continuous helical lesion around a complete or nearly complete turn of the spiral/helix. In some embodiments, the wire electrode 406 can have a diameter of between about 0.002 inches and about 0.010 inches (e.g., about 0.008 inches). In other embodiments, the therapeutic assembly 404 can include a "ground" electrode that is electrically insulated from the spiral at a more proximal portion of the spiral/helix (e.g., a bipolar configuration). The spiral/helix can have a constant diameter, or in other embodiments the spiral/helix can have a varying diameter. For example, spiral/helix can have a diameter that tapers in a distal direction or a proximal direction. In other embodiments, the single wire electrode has discrete dielectric coating segments that are spaced apart from each other to define discrete energy delivery elements between the dielectric coating segments. The single wire electrode can be made from a shape memory metal or other suitable material. Additionally, the control algorithm 140 (FIG. 1) can be adjusted to account for the increased surface area contact of the single wire electrode 406 such that sufficient ablation depths can be achieved without charring or overheating the inner wall of the vessel.

In some embodiments, the single wire electrode 406 can be delivered with the guide catheter (not shown) or an additional sheath (not shown) for precise positioning and deployment. The guide catheter (not shown) can be advanced and/or manipulated until positioned at a desired location proximate the treatment site. The therapeutic assembly 404 can then be inserted through the guide catheter. In some embodiments, the therapeutic assembly 404 expands into a helical/spiral shape immediately once exiting a distal end of the guide catheter. In other embodiments, the single wire electrode 406 can be tubular and transforms into a helical/spiral shape when a guide wire (placed therethrough) is removed in a proximal direction.

### A. Rotation Devices and Methods

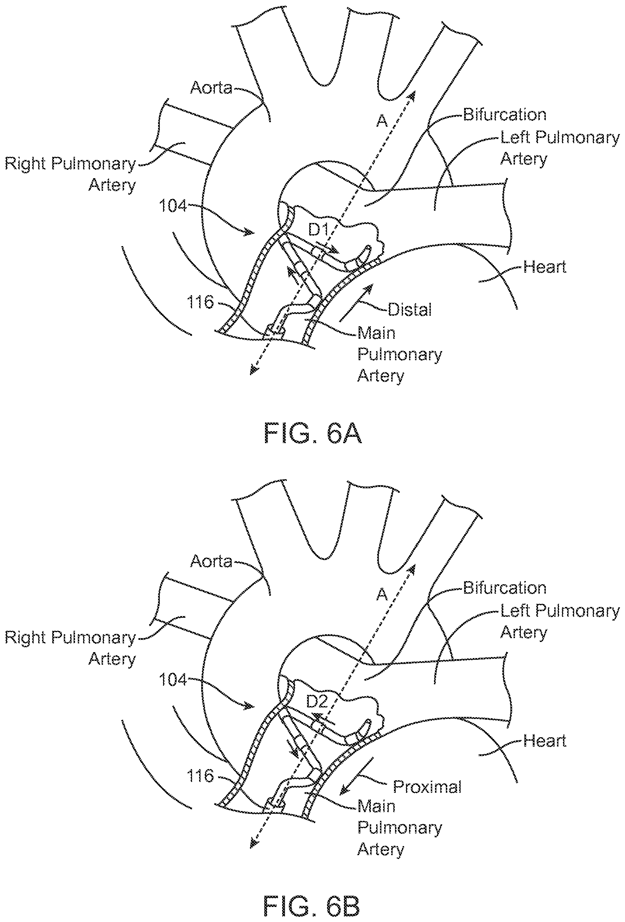

As shown in FIGS. 6A and 6B, the therapeutic assembly 104 can be configured to rotate about a longitudinal axis A when advanced distally from the shaft 116 or retracted proximally from the shaft 116. For example, when the therapeutic assembly 104 is advanced distally, the spiral/helical structure can rotate in a first direction D1 (FIG. 6A). Likewise, when the therapeutic assembly 104 is retracted proximally, the spiral/helical structure can rotate in a second direction D2 (FIG. 6B). Such a rotational feature can be particularly advantageous in the pulmonary vessels, since, at least at the MPA and proximal portions of the LPA and RPA, the pulmonary vessels have relatively large diameters that can require a large number of lesions to provide fully-circumferential coverage and/or effective treatment. To compensate for this, effective treatment in the pulmonary vessels can often times require multiple rotations of the therapeutic assembly 104 to reposition the therapeutic assembly 104 and achieve such a fully-circumferential lesion. Additionally, rotation of the therapeutic assembly 104 can aid in maneuvering the therapeutic assembly 104 through a turn in a vessel, such as when accessing a branch or segment of a larger vessel (e.g., accessing the LPA and RPA from the MPA).

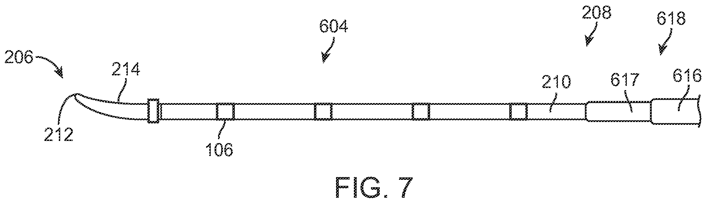

FIG. 7 is a side view of another embodiment of a catheter configured in accordance with the present technology. The catheter can include a therapeutic assembly 604 generally similar to the previously described therapeutic assembly 104 (referenced herein with respect to FIGS. 1-4). As shown in FIG. 7, the catheter includes an inner sheath 617 slidably positioned within a guide catheter 616 between the guide catheter 616 and the therapeutic assembly 604. In certain vessels, contact forces between the therapeutic assembly 604 and the vessel wall can make it difficult to rotate the therapeutic assembly 604 distally and/or proximally. Likewise, a catheter and/or a sheath carrying the catheter may be insufficiently flexible to match the curvature of anatomy near the treatment location, such as the curvature of a pulmonary artery between the MPA and the RPA and/or LPA. This may cause the catheter and/or the sheath to enter the body lumen out of alignment with a longitudinal axis of the body lumen. Because of the inner sheath 617 of the present technology, the guide catheter 616 and the inner sheath 617 can rotate along a central axis independently of one another. Moreover, the inner sheath 617 can be sufficiently flexible to de-couple at least the therapeutic assembly 604 (positioned within a relatively stable pulmonary vessel) from the catheter (e.g., the guide catheter 616) positioned within or nearer to the contracting and expanding heart. This feature can be advantageous because, for example, when at least a portion of the catheter and/or shaft is positioned within the heart, the guide catheter 616 often time translates the pumping movement of the heart to the therapeutic assembly 604.

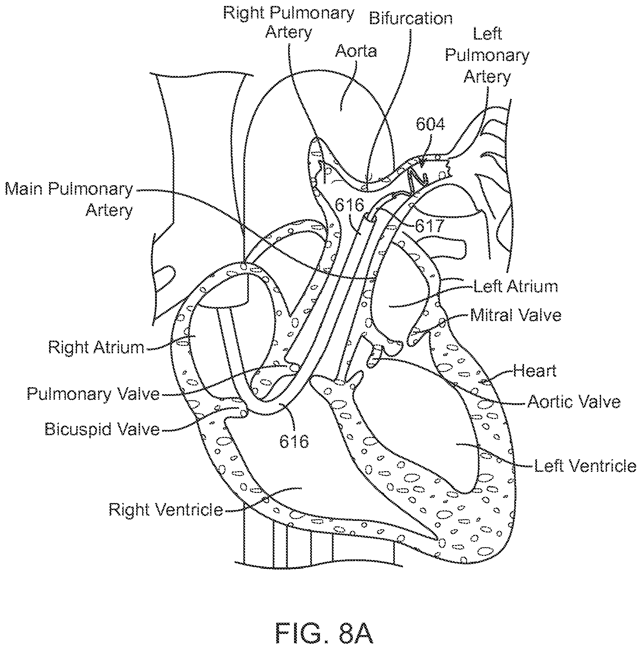

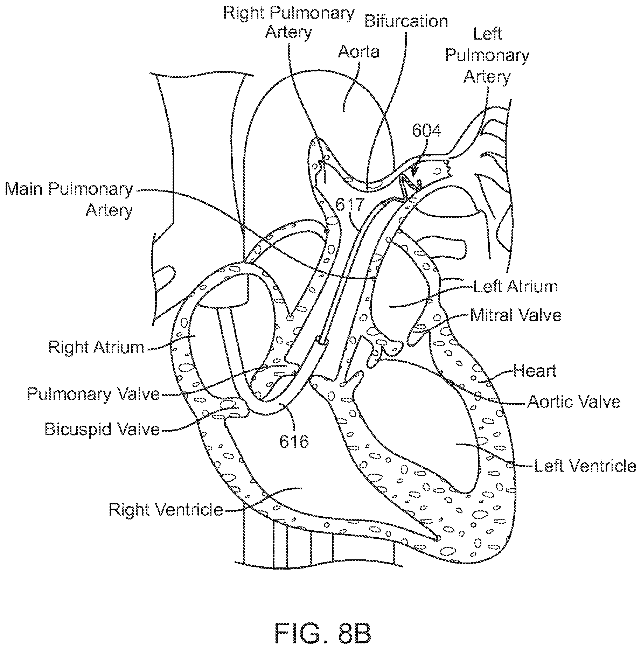

FIGS. 8A and 8B show examples of various deployment configurations of the catheter with the inner sheath 617. As shown in FIG. 8A, the shaft 616 can be advanced along the MPA just proximal to the ostium of the LPA (or RPA (not shown)). The inner sheath 617 (containing the therapeutic assembly 604) can then be advanced past the distal end of the shaft 616 and into the LPA for deployment of the therapeutic assembly 604. As shown in FIG. 8B, in some embodiments the shaft 616 can be advanced just distal of the pulmonary valve. The inner sheath 617 can then be advanced past the distal end of the shaft 616, past the bifurcation, and into the LPA for deployment of the therapeutic assembly 604.

### B. Anchoring Devices and Methods

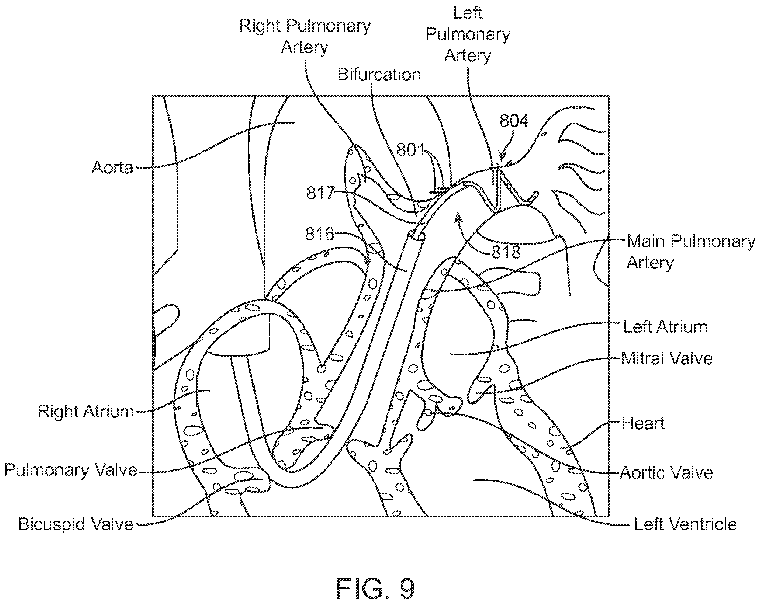

FIG. 9 is a side view of another embodiment of a catheter shown in the deployed configuration within the LPA in accordance with the present technology. The catheter can be generally similar to the previously described catheters 110 or (referenced herein with respect to FIGS. 1-7A). However, as shown in FIG. 9, the catheter includes fixation members 801 (shown schematically for illustrative purposes only) along at least a portion of its shaft 816 and/or inner sheath 817. The fixation members 801 can be configured to contact the inner wall of the pulmonary vessel and stabilize the distal portion 818 and/or therapeutic assembly 804 with respect to the pulmonary vessel. Such stabilization can be advantageous because the pulmonary vessels constantly move as a result of the surrounding anatomy, particularly the contraction and relaxation of the heart, and also the respiratory cycle. As previously discussed, the most common intravascular approach to the pulmonary vessel involves the positioning of at least a portion of the catheter and/or shaft within the heart. As a result, the shaft translates the pumping movement of the heart to the therapeutic assembly 804. The fixation members 801can stabilize at least the therapeutic assembly 804 within the pulmonary vessel so that movement of the catheter (e.g., the shaft 816) will not affect the alignment and/or contact of the therapeutic assembly 804 and the vessel wall. In some embodiments, the fixation members 801 can be atraumatic or non-tissue penetrating, and in other embodiments the fixation members 801 can be tissue-penetrating (e.g., embedded in the tissue by radial force). The fixation members 801 can have any size or configuration suitable to stabilize the therapeutic assembly 804 relative to the vessel.

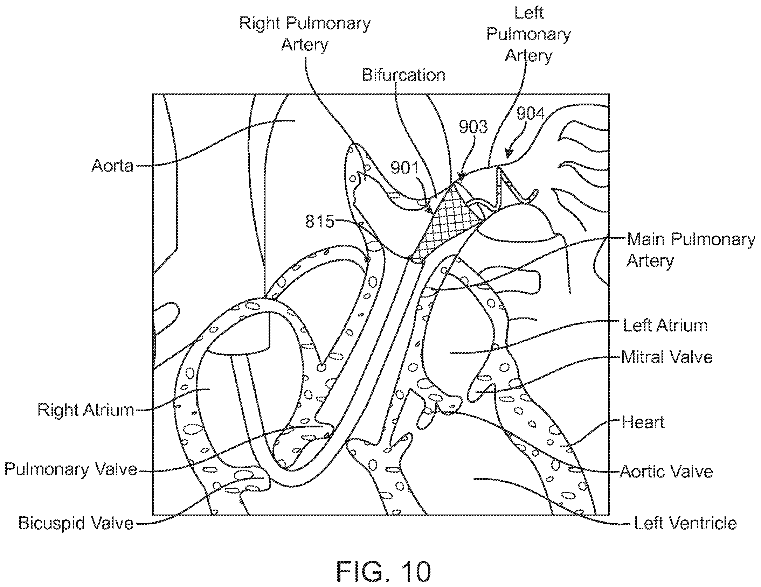

FIG. 10 is a side view of another embodiment of a catheter shown in the deployed configuration within the LPA in accordance with the present technology. The catheter can include an expandable inner sheath 901 that, when in the deployed configuration, expands to an outer radius generally equal to or greater than the inner radius of the vessel at the target location (e.g., a pulmonary vessel). As such, at least a distal end 903 of the sheath 901 can expand to engage the vessel wall thereby exerting a radially outward force against the vessel wall and stabilizing the sheath 901. In some embodiments, the sheath 901 can comprise an expandable stent-like structure which is collapsed in a delivery state within the elongated shaft 916 and expanded to a deployed state when advanced beyond a distal end 915 of the elongated shaft 916. Once deployed, the sheath 901 helps to mechanically isolate the therapeutic assembly 904 from the shaft 916. The sheath 901 can have a generally tapered shape such that the distal end 903 of the sheath 901 has a greater diameter than a proximal end (not shown). In some embodiments, at least a portion of the sheath 901 can include one or more fixation members configured to engage the vessel wall.

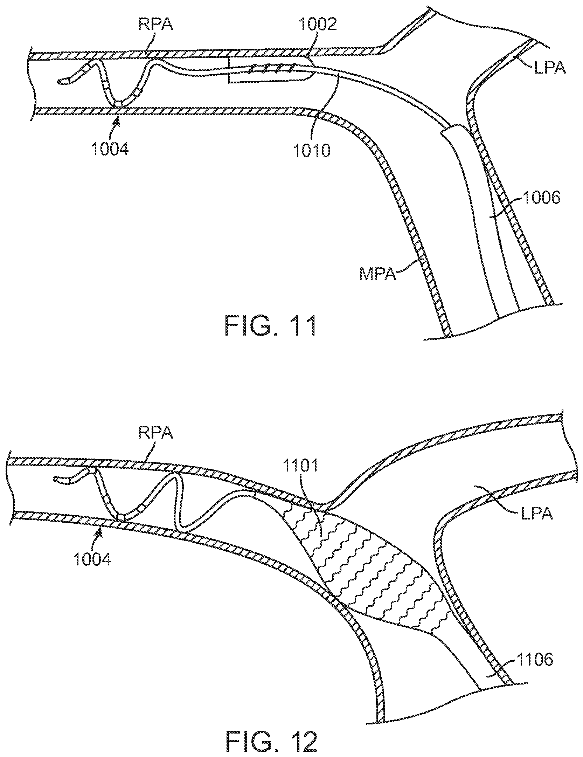

FIG. 11 is a side view of another embodiment of a catheter shown in the deployed configuration within the RPA in accordance with the present technology. The catheter can include a guide sheath 1006 and a circumferentially grooved or threaded elongated member 1010 slideably positioned therethrough. As shown in FIG. 11, the elongated member 1010 can be mated with an anchor 1002. Once deployed, the anchor 1002 can be fixed or secured to the vessel wall by frictional force and/or fixation members (not shown) (see FIG. 9 and accompanying description). In operation, insertion of the catheter 1017 from its proximal end (not shown) causes the therapeutic assembly 1004 to rotate in a distal direction while the anchor 1002 remains relatively generally stationary. In some embodiments (not shown), the anchor 1002 can be fixed to the guide sheath 1006.

FIG. 12 is a side view of another embodiment of a catheter shown in the deployed configuration within the RPA in accordance with the present technology. The catheter can include an expandable anchor 1101 configured to expand against at least a portion of the vessel wall and secure the therapeutic assembly 1104 relative to the local anatomy. For example, as shown in FIG. 12, once advanced distally past the catheter shaft 1106, the expandable anchor 1101 can expand and exert an outward force against the vessel wall. In particular embodiments, the anchor 1101 can engage and/or exert a contact force in one or more branches of the pulmonary artery simultaneously. For example, as shown in the illustrated embodiment, the anchor 1101 can span the bifurcation of the MPA into the LPA and/or RPA. Additionally, the anchor 1101 can have a tapered shape in the proximal and/or distal directions, and in other embodiments, the anchor 1101 can have a relatively uniform cross-sectional area along its length. In yet other embodiments, the anchor 1101 can have a main body and one or more branches (not shown) configured to be positioned within at least a portion of the MPA and the LPA or RPA, respectively. In some embodiments, the expandable anchor 1101 can be a stent, balloon, self-expanding basket or other suitable expandable or shape-changing structures or devices.

### C. Tension-Relieving Devices and Methods

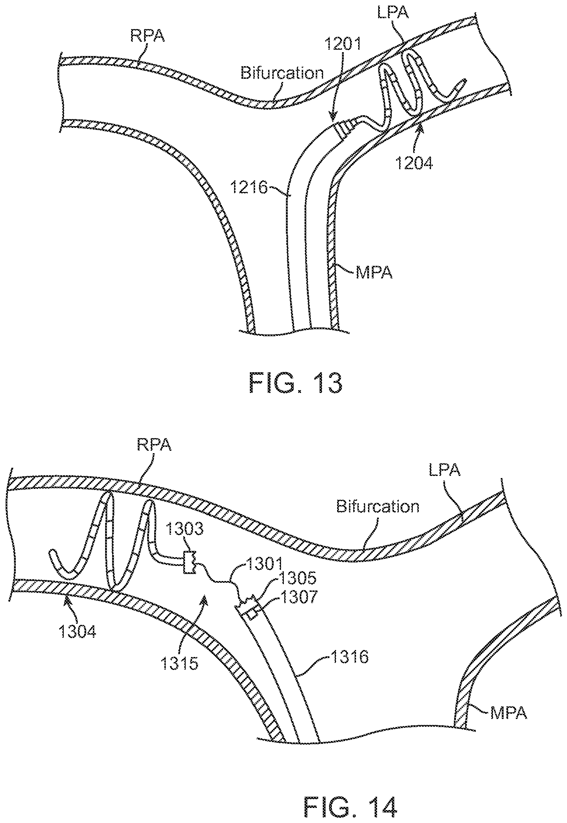

FIG. 13 is a side view of another embodiment of the catheter having a collapsible inner shaft 1201 configured in accordance with an embodiment of the present technology. At least a proximal portion of the therapeutic assembly 1204 can be carried by the inner shaft 1201. As shown in FIG. 13, the inner shaft 1201 can have a "telescoping" design that allows the inner shaft 1201 to extend and retract freely such that proximal and distal movement of the shaft 1216 caused by the cardiac cycle, respiration, etc. will not pull or push the therapeutic assembly 104 out of position. Instead such motion is absorbed by the collapsible/extendable design of the inner shaft 1201. In some embodiments, the catheter can include a locking and/or activation mechanism (not shown) so that the timing and/or extent of the extension/retraction of the inner shaft 1201 can be controlled by the clinician. In further embodiments, the inner shaft can be corrugated along at least a portion of length to allow extension and retraction. Likewise, in a particular embodiment, the inner shaft 1201 can be a braided structures having a plurality of sections with alternating flexibility (e.g., by altering wire diameter, wire count, etc.) As a result, the sectioned inner shaft 1201 would allow for compression and extension with motion, thus mechanically isolating (at least in part) the therapeutic assembly 1204 from the shaft 1206.

FIG. 14 is a side view of another embodiment of the catheter having a therapeutic assembly 1304 mechanically isolated from the shaft 1316 by an isolating element 1315. The isolating element 1315 can include a first portion 1303 operably connected to the therapeutic assembly 1304, a second portion 1305 operably connected to the shaft 1316, and a connector 1301 therebetween. The connector 1301 can have enough slack such that the position of the therapeutic assembly 1304 with respect to the vessel in which it is expanded is generally unaffected by movement of the shaft 1316. As discussed above, often times during cardiac contraction and relaxation the movement of the shaft 1316 is strong enough to pull or push the therapeutic assembly 1304 along the pulmonary vessel. For example, when the heart contracts, the shaft 1316 can be pulled distally by the contracting heart muscles, thereby pulling the therapeutic assembly 1304 distally (and likely out of position). The isolating element 1315 of the present technology mechanically isolates the therapeutic assembly 1304 from the catheter shaft 1316, allowing the shaft to move while the therapeutic assembly 1304 remains relatively stationary. In some embodiments, the catheter can include a locking and/or activation mechanism 1307 operably connected to the isolating member 1315 so that the timing of the release of the therapeutic assembly 1304 from the shaft 1316 can be controlled by the clinician. Additional devices and deployment methods for mechanical isolation of the therapeutic assembly from the shaft and/or catheter can be found in U.S. Patent Application No. 13/836,309, filed March 15, 2013, titled "CATHETERS HAVING TETHERED NEUROMODULATION UNITS AND ASSOCIATED DEVICES, SYSTEMS, AND METHODS".

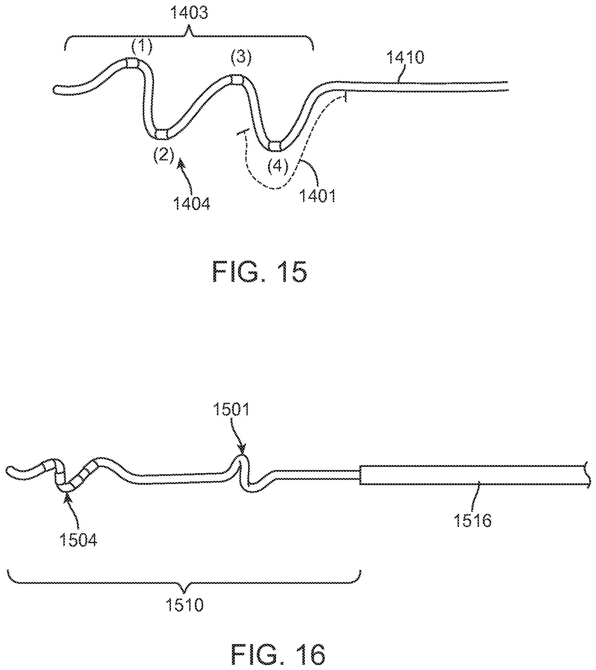

In some embodiments, the therapeutic assembly and/or support structure can be modified to relieve tension between therapeutic assembly and the shaft. For example, as shown in FIG. 14, the support structure 1410 can include an extended segment 1401 at a proximal section of the helical/spiral portion 1403 of the support structure 1410 and/or therapeutic assembly 1404. Such an extension can provide more slack and greater flexibility at the proximal section of the helical/spiral portion 1403. Additionally, one or more turns (labeled (1), (2), (3) and (4) in FIG. 14) can be added to the support structure 1410 to increase flexibility and/or the lengthening potential of the therapeutic assembly 1404. In a particular embodiment shown in FIG. 16, an inflection section 1501 can be included along the generally straight portion of the support structure 1510. Similar to the features described above with reference to FIG. 15, the inflection section 1501 can provide the added slack to absorb the disruptive motion of the shaft 1516.

### IV. Conclusion

The above detailed descriptions of embodiments of the technology are not intended to be exhaustive or to limit the technology to the precise form disclosed above. Although specific embodiments of, and examples for, the technology are described above for illustrative purposes, various equivalent modifications are possible within the scope of the technology, as those skilled in the relevant art will recognize. For example, while steps are presented in a given order, alternative embodiments may perform steps in a different order. The various embodiments described herein may also be combined to provide further embodiments.

From the foregoing, it will be appreciated that specific embodiments of the technology have been described herein for purposes of illustration, but well-known structures and functions have not been shown or described in detail to avoid unnecessarily obscuring the description of the embodiments of the technology. Where the context permits, singular or plural terms may also include the plural or singular term, respectively.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. The present invention is set out in the appended claims. The embodiments, aspects or examples according to the present description that do not fall within the scope of said claims are provided for illustrative purposes only and do not form part of the present invention.

## Claims

1. A catheter apparatus (110), comprising:
an elongated shaft (116, 1306) having a proximal portion (114) and a distal portion (118), wherein the distal portion (118) of the shaft (116, 1306) is configured for intravascular delivery to a body vessel of a human patient;
a therapeutic assembly (104, 1304) at the distal portion (118) of the elongated shaft (116, 1306) comprising a pre-formed shape, and wherein the therapeutic assembly (104, 1304) is transformable between-
a substantially straight delivery configuration; and
a treatment configuration having the pre-formed helical shape to position the therapeutic assembly (104, 1304) in stable contact with a wall of the body vessel;
an isolating element (1315) including a first portion (1303) operably connected to the therapeutic assembly (104, 1304), a second portion (1305) operably connected to the shaft (116, 1306), and a connector (1301) therebetween, wherein the isolating element (1315) is configured to allow the shaft (116, 1306) to move while the therapeutic assembly (104, 1304) remains stationary ; and
a locking and/or activation mechanism (1307) operably connected to the isolating element (1315) so that the release of the therapeutic assembly (104, 1304) from the shaft (116, 1306) can be controlled,
wherein the distal portion (118) of the elongated shaft (116, 1306) and the therapeutic assembly (104, 1304) are sized and configured for intravascular delivery into the pulmonary artery.

2. The catheter apparatus (110) of claim 1 wherein the therapeutic assembly (104, 1304) comprises a pre-formed helical member defined by a single wire electrode.

3. The catheter apparatus (110) of claim 1, further including a plurality of energy delivery elements (106) carried by the therapeutic assembly (104, 1304).

4. The catheter apparatus (110) of claim 1 wherein the isolating element (1315) is configured to mechanically isolate the therapeutic assembly (104, 1304) from the shaft (116, 1306).

5. The catheter apparatus (110) of claim 1, comprising:
an inner sheath within the elongated shaft (116, 1306) and separating at least a portion of the elongated shaft (116, 1306) from the therapeutic assembly (104, 1304).

6. The catheter apparatus (110) of claim 5, wherein at least a portion of the inner sheath is configured to expand and exert a radially outward force on the vessel wall.

7. System (100) comprising the catheter apparatus (110) according to any of the preceding claims and a generator (132).

8. System (100) according to claim 7, wherein the catheter apparatus (110) and the generator (132) are connected with each other.

## Patentansprüche

1. Kathetervorrichtung (110), umfassend:
einen länglichen Schaft (116, 1306), der einen proximalen Abschnitt (114) und einen distalen Abschnitt (118) aufweist, wobei der distale Abschnitt (118) des Schaftes (116, 1306) zur intravaskulären Abgabe an ein Körpergefäß eines menschlichen Patienten konfiguriert ist;
eine therapeutische Anordnung (104, 1304) an dem distalen Abschnitt (118) des länglichen Schaftes (116, 1306), umfassend eine vorgeformte Form, und wobei die therapeutische Anordnung (104, 1304) transformierbar ist zwischen -
einer im Wesentlichen geraden Abgabekonfiguration; und
einer Behandlungskonfiguration, die die vorgeformte spiralförmige Form aufweist, um die therapeutische Anordnung (104, 1304) in stabilem Kontakt mit einer Wand des Körpergefäßes auszurichten;
ein Trennelement (1315), das einen ersten Abschnitt (1303) einschließt, der funktionell mit der therapeutischen Anordnung (104, 1304) verbunden ist, einen zweiten Abschnitt (1305), der funktionell mit dem Schaft (116, 1306) verbunden ist, und einen Verbinder (1301) dazwischen, wobei das Trennelement (1315) konfiguriert ist, um es dem Schaft (116, 1306) zu ermöglichen, sich zu bewegen, während die therapeutische Anordnung (104, 1304) feststehend bleibt; und
ein Verriegelungs- und/oder Aktivierungsmechanismus (1307), der funktionell mit dem Trennelement (1315) verbunden ist, so dass die Freisetzung der therapeutischen Anordnung (104, 1304) von dem Schaft (116, 1306) gesteuert werden kann,
wobei der distale Abschnitt (118) des länglichen Schaftes (116, 1306) und die therapeutische Anordnung (104, 1304) für eine intravaskuläre Abgabe in die Lungenarterie dimensioniert und konfiguriert sind.

2. Kathetervorrichtung (110) nach Anspruch 1, wobei die therapeutische Anordnung (104, 1304) ein vorgeformtes schraubenförmiges Element umfasst, das durch eine einzelne Drahtelektrode definiert ist.

3. Kathetervorrichtung (110) nach Anspruch 1, die ferner eine Vielzahl von Energiezuführelementen (106) einschließt, die von der therapeutischen Anordnung (104, 1304) getragen werden.

4. Kathetervorrichtung (110) nach Anspruch 1, wobei das Trennelement (1315) konfiguriert ist, um die therapeutische Anordnung (104, 1304) mechanisch von dem Schaft (116, 1306) zu isolieren.

5. Kathetervorrichtung (110) nach Anspruch 1, umfassend:
eine innere Hülse innerhalb des länglichen Schaftes (116, 1306), die mindestens einen Abschnitt des länglichen Schaftes (116, 1306) von der therapeutischen Anordnung (104, 1304) trennt.

6. Kathetervorrichtung (110) nach Anspruch 5, wobei mindestens ein Abschnitt der inneren Hülse konfiguriert ist, um sich auszudehnen und eine radial nach außen gerichtete Kraft auf die Gefäßwand auszuüben.

7. System (100), umfassend die Kathetervorrichtung (110) nach einem der vorstehenden Ansprüche und einen Generator (132).

8. System (100) nach Anspruch 7, wobei die Kathetervorrichtung (110) und der Generator (132) miteinander verbunden sind.

## Revendications

1. Appareil de cathéter (110), comprenant :
une tige allongée (116, 1306) ayant une partie proximale (114) et une partie distale (118), dans lequel la partie distale (118) de la tige (116, 1306) est configurée pour une libération intravasculaire à un vaisseau corporel d'un patient humain ;
un ensemble thérapeutique (104, 1304) au niveau de la partie distale (118) de la tige allongée (116, 1306) comprenant une forme préformée, et dans lequel l'ensemble thérapeutique (104,1304) est transformable entre-
une configuration de libération essentiellement linéaire ; et
une configuration de traitement ayant la forme hélicoïdale préformée pour positionner l'ensemble thérapeutique (104, 1304) en contact stable avec une paroi du vaisseau corporel ;
un élément isolant (1315) incluant une première partie (1303) reliée opérationnellement à l'ensemble thérapeutique (104, 1304), une deuxième partie (1305) reliée opérationnellement à la tige (116, 1306), et un connecteur (1301) entre eux, dans lequel l'élément isolant (1315) est configuré pour permettre à la tige (116, 1306) de bouger alors que l'ensemble thérapeutique (104,1304) demeure stationnaire ; et
un mécanisme de verrouillage et/ou d'activation (1307) relié opérationnellement à l'élément isolant (1315) de sorte que la libération de l'ensemble thérapeutique (104, 1304) de la tige (116, 1306) peut être commandée,
dans lequel la partie distale (118) de la tige allongée (116, 1306) et l'ensemble thérapeutique (104, 1304) sont dimensionnés et configurés pour une libération intravasculaire dans l'artère pulmonaire.

2. Appareil de cathéter (110) selon la revendication 1 dans lequel l'ensemble thérapeutique (104, 1304) comprend un élément hélicoïdal préformé défini par une électrode à fil unique.

3. Appareil de cathéter (110) selon la revendication 1, incluant en outre une pluralité d'éléments de libération d'énergie (106) portés par l'ensemble thérapeutique (104,1304).

4. Appareil de cathéter (110) selon la revendication 1 dans lequel l'élément isolant (1315) est configuré pour isoler mécaniquement l'ensemble thérapeutique (104, 1304) de la tige (116, 1306).

5. Appareil de cathéter (110) selon la revendication 1, comprenant :
une gaine interne au sein de la tige allongée (116, 1306) et séparant au moins une partie de la tige allongée (116, 1306) de l'ensemble thérapeutique (104, 1304).

6. Appareil de cathéter (110) selon la revendication 5, dans lequel au moins une partie de la gaine interne est configurée pour s'expanser et exercer une force radialement vers l'extérieur sur la paroi de vaisseau.

7. Système (100) comprenant l'appareil de cathéter (110) selon l'une quelconque des revendications précédentes et un générateur (132).

8. Système (100) selon la revendication 7, dans lequel l'appareil de cathéter (110) et le générateur (132) sont reliés l'un à l'autre.
